# EUROPEAN PATENT APPLICATION

(11) **EP 3 640 640 A1**
(43) Date of publication of application: **22.04.2020**
(21) Application number: 18817821.4
(22) Date of filing: 09.04.2018
(51) Int. Cl.: G01N 33/48

(54) **BLOOD INSPECTION DEVICE**

(30) Priority: 13.06.2017 JP 2017115891
(71) Applicant: Konica Minolta, Inc., Tokyo 100-7015 (JP)
(72) Inventor: MAJIMA, Masanao, Tokyo 100-7015 (JP)
(74) Representative: Gille Hrabal
(86) International application number: PCT/JP2018/014902
(87) International publication number: WO 2018/230118

(57) **Abstract**

This blood inspection device is for inspecting blood in a blood collection tube. The blood inspection device comprises an output unit for outputting, to the outside, first information about the anticoagulants that can and cannot be used for each inspection item.

## Description

### Technical Field

The present invention relates to a blood testing device.

### Background Art

In recent years, blood testing devices capable of conducting tests on one or more test items have come to be used for blood testing. Blood testing using a blood testing device involves taking a blood sample from a subject using blood collection tube and the like and dispensing the blood sample into a test cartridge. Then, the test cartridge into which the blood sample is dispensed is set in the blood testing device and a blood test is conducted.

In conducting a blood test, an anticoagulant is enclosed in the blood collection tube beforehand from the viewpoint of preventing the blood sample from coagulating. Also, a usable anticoagulant, that is, an anticoagulant that does not affect the test results, varies with the test item. If a blood test is conducted using a blood sample doped with an anticoagulant that might affect test results, proper results may not be available. In this case, if none of doctors, nurses, and the like notices the mistake in the test results, wrong treatment might be administered. Generally, users such as medical technologists conduct blood tests by checking test items and anticoagulants usable for the test items using a list on paper, a laboratory test information system, or the like.

Also, Patent Literature (hereinafter abbreviated as PTL) 1 describes a method for identifying an anticoagulant contained in a blood sample used for testing. The method described in PTL 1 identifies the anticoagulant in the blood sample in parallel with the blood test or after the blood test.

### Citation List

### Patent Literature

PTL 1
Japanese Unexamined Patent Application Publication (Translation of PCT Application) No. 2011-508223

### Summary of Invention

### Technical Problem

However, with the method of PTL 1, since the anticoagulant is identified in parallel with the blood test or after the blood test, test results of the blood test may be wasted. Also, even if the user checks combinations of anticoagulants and test items against the list on paper, the laboratory test information system, or the like, the user may conduct a blood test using a blood sample doped with an anticoagulant that might affect test results due to a misreading, misunderstanding, or the like.

Thus, an object of the present invention is to provide a blood testing device, where in conducting a blood test using a blood sample in a blood collection tube, the blood testing device can prevent a blood sample doped with an anticoagulant that might affect test results from being used in the test.

### Solution to Problem

To achieve at least one of the abovementioned objects, according to an aspect of the present invention, a blood testing device reflecting one aspect of the present invention is a device for testing blood in a blood collection tube, the blood testing device comprising an output that externally outputs first information about usable anticoagulants or unusable anticoagulants for respective test items.

### Advantageous Effects of Invention

The present invention can provide a blood testing device that can prevent users from mistakenly testing blood containing an anticoagulant that might affect test results.

### Brief Description of Drawings

FIG. 1 is a perspective view schematically showing a blood testing device according to an embodiment of the present invention.
FIG. 2 is a block diagram of the blood testing device.
FIG. 3 is a schematic diagram showing an exemplary presentation on a touch panel display.
FIG. 4 is a schematic diagram showing another exemplary presentation on the touch panel display.
FIG. 5 is a schematic diagram showing another exemplary presentation on the touch panel display.

### Description of Embodiments

A blood testing device according to an embodiment of the present invention will be described below. The blood testing device according to the embodiment of the present invention is used to detect substances-to-be-detected contained in a subject's blood sample collected using a blood collection tube in which an anticoagulant is enclosed.

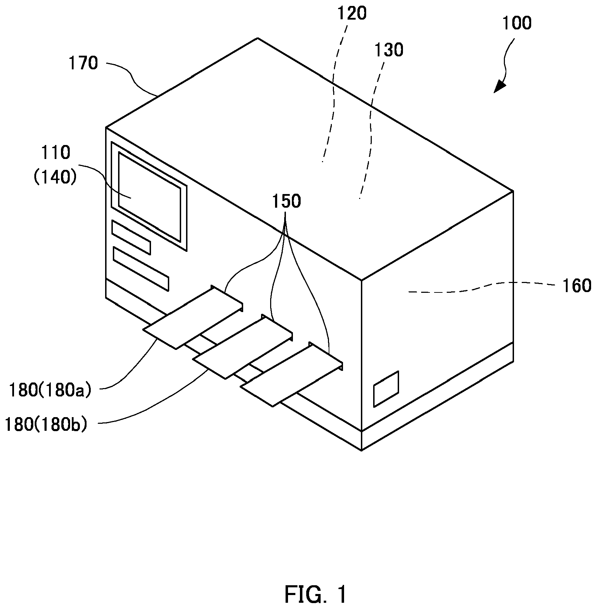

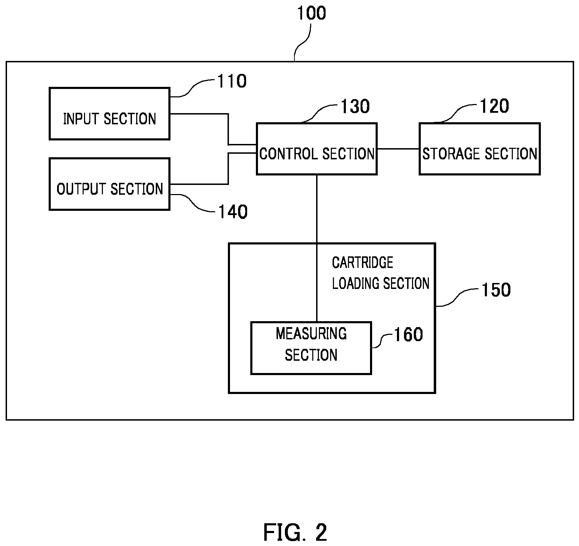

FIG 1 is a schematic diagram showing a configuration of blood testing device 100. FIG. 2 is a block diagram of blood testing device 100. As shown in FIGS. 1 and 2, blood testing device 100 includes output section 140 that externally outputs first information about anticoagulants usable for respective test items. Also, according to the present embodiment, blood testing device 100 further includes input section 110 used to enter second information about an anticoagulant contained in a blood collection tube, storage section 120 that stores the first information, control section 130 that determines whether the anticoagulant contained in the blood collection tube is usable for testing a specific test item, cartridge loading section 150 into which test cartridge 180 is loaded, and measuring section 160 that conducts blood testing. Input section 110, control section 130, output section 140, cartridge loading section 150, and measuring section 160 are housed in casing 170.

Input section 110 is used by a user to enter the second information about the type of anticoagulant contained in the blood collection tube. A configuration of input section 110 can be selected as appropriate. Input section 110 may be a keyboard, a bar code reader, plural buttons provided for respective types of anticoagulants, or a touch panel display serving also as output section 140. According to the present embodiment, input section 110 is a touch panel display serving also as output section 140. Also, input section 110 may be used to enter not only the first information, but also a specimen ID or the like.

Herein, examples of the anticoagulant contained in the blood collection tube include EDTA dipotassium salt, EDTA tripotassium salt, EDTA disodium salt, trisodium citrate (9:1), trisodium citrate (4:1), fluoride/EDTA, fluoride/oxalate, fluoride/heparin, heparin lithium, heparin sodium, citrate, phosphate, glucose, and adenine.

Also, the "second information" contains information about an anticoagulant, examples of which include a name of the anticoagulant, a character code corresponding to the anticoagulant, a color code corresponding to the anticoagulant and marked on a cap of the blood collection tube, and combinations thereof. Also, the color code may be a color code defined by JIS T 3233, ISO 6710, or CLSI GP41-A6 or a color code set arbitrarily for the anticoagulant.

The second information may be recorded as character information or as a bar code on the blood collection tube. Also, the bar code may be a one-dimensional bar code or a two-dimensional bar code. Note that when the second information is recorded as a bar code on the blood collection tube, the input section 110 described above is a bar code reader.

Table 1 shows examples of the name of the anticoagulant contained in the blood collection tube, the character code corresponding to the anticoagulant contained in the blood collection tube, and the JIS T 3233-based color code on the cap of the blood collection tube corresponding to the anticoagulant contained in the blood collection tube.

### [Table 1]

**Table 1**

| Anticoagulant | Character code | Color code |
|---|---|---|
| EDTA dipotassium salt | K2E | Lavender |
| EDTA tripotassium salt | K3E | Lavender |
| EDTA disodium salt | N2E | Lavender |
| Trisodium citrate (9:1) | 9NC | Light blue |
| Trisodium citrate (4:1) | 4NC | Black |
| Fluoride/oxalate | FX | Gray |
| Fluoride/EDTA | FE | Gray |
| Fluoride/heparin | FH | Green |
| Heparin lithium | LH | Green |
| Heparin sodium | NH | Green |
| Citrate, phosphate, glucose, adenine | CPDA | Yellow |
| - | Z | Red |

Storage section 120 stores the first information about the anticoagulants usable for respective test items. For example, the first information includes information that anticoagulant a and anticoagulant b are usable for test item A and that only anticoagulant c is usable for test item B. In this way, since each test item is associated with the anticoagulant(s) usable for testing the test item, control section 130 can appropriately determine the anticoagulant(s) usable for the test as described later.

Control section 130 compares the second information entered via input section 110 with the first information stored in storage section 120, and determines whether the anticoagulant contained in the blood collection tube is usable for testing a specific test item. Specifically, if the comparison between the first information and the second information reveals that the anticoagulant is unusable for the test item, control section 130 performs control to make the test item in question unselectable, notifies that the test item in question cannot be tested, or performs control to disable the test item in question from being tested. Also, when the user intends to test plural test items, if the anticoagulant is unusable for one or more of the plural test items, control section 130 performs control to make the test item(s) in question unselectable, notifies that the test item(s) in question cannot be tested, or performs control to disable the test item(s) in question from being tested.

Output section 140 externally outputs the first information or externally outputs the first information and a determination result produced by control section 130. Output section 140 may output the first information, or the first information and determination result as visual information including at least either characters or graphics, as auditory information including voice information, or as both visual information and auditory information. Output section 140 may output information other than the first information or the first information and determination result. For example, output section 140 may also output information entered into input section 110 or information about the progress of testing. A configuration of output section 140 can be selected as appropriate as long as output section 140 can output at least either of visual information and auditory information. Examples of output section 140 that outputs visual information include a display. Also, examples of output section 140 that outputs auditory information include a speaker. According to the present embodiment, output section 140 is a touch panel display serving also as input section 110.

Test cartridge 180 used in testing is loaded into cartridge loading section 150. The number of cartridge loading sections 150 can be set as appropriate, and may be one or more than one. According to the present embodiment, the number of cartridge loading sections 150 is three.

Test cartridge 180 includes a reaction section that causes a blood sample and test reagent to react with each other. Also, test cartridge 180 may contain a test reagent for use in blood testing. According to the present embodiment, test cartridge 180 includes the reaction section and houses a test reagent corresponding to the test item. Also, test cartridge 180 may be labeled with test items testable using the test cartridge and information about anticoagulants that do not affect test results of the test items.

Measuring section 160 conducts blood testing. A configuration of measuring section 160 can be set as appropriate as long as substances-to-be-detected contained in the blood sample can be detected. For example, measuring section 160 may detect substances-to-be-detected in the blood sample using an antigen-antibody reaction and surface plasmon resonance fluorescence spectroscopy (Surface Plasmon-field enhanced Fluorescence Spectroscopy: SPFS).

Casing 170 houses input section 110, control section 130, output section 140, cartridge loading section 150 and measuring section 160. According to the present embodiment, input section 110 and output section 140 are placed on a surface of casing 170. Also, openings of cartridge loading sections 150 are formed in a surface of casing 170 to load test cartridges 180 into cartridge loading sections 150. Also, control section 130 and measuring section 160 are housed in casing 170.

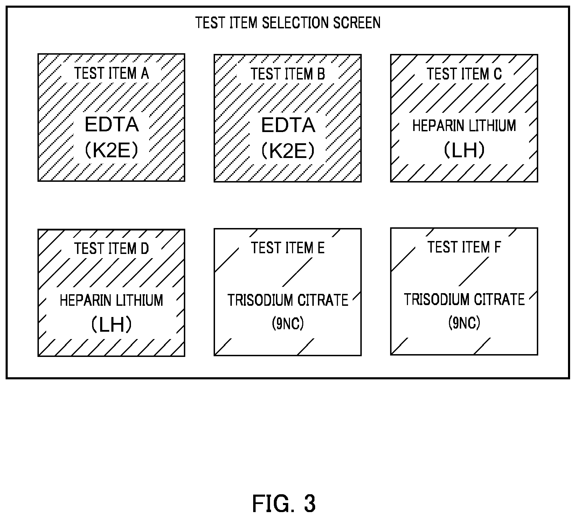

Now, operation of blood testing device 100 will be described. FIGS. 3 to 5 are schematic diagrams showing exemplary presentations on a touch panel display that combines input section 110 and output section 140.

### (First aspect)

A first aspect will be described with reference to FIG. 3. In the first aspect, first, test cartridge 180 corresponding to a test item is set in cartridge loading section 150 of blood testing device 100. Next, the user dispenses a blood sample into test cartridge 180. Note that test cartridge 180 may be set in cartridge loading section 150 after the blood sample is dispensed into test cartridge 180.

The touch panel display that combines input section 110 and output section 140 displays the first information. As shown in FIG 3, according to the present embodiment, test items A to F as well as the names of anticoagulants usable for the respective test items are each displayed on the touch panel display and the test items are color-coded by anticoagulant. In FIG. 3, the color-coding of the anticoagulants is indicated by hatch patterns.

The user compares the second information about the anticoagulant contained in the blood collection tube with the first information displayed on the touchup display and enters the test item to be tested. Specifically, by touching the area of the touch panel display in which the test item is displayed, the user checks that the second information matches the first information stored in storage section 120.

In this way, in the example of FIG. 3, since test items and anticoagulants usable for the respective test items are shown on the touch panel display, the user can prevent a blood sample containing anticoagulants likely to affect test results from being dispensed from the blood collection tube.

### (Second aspect)

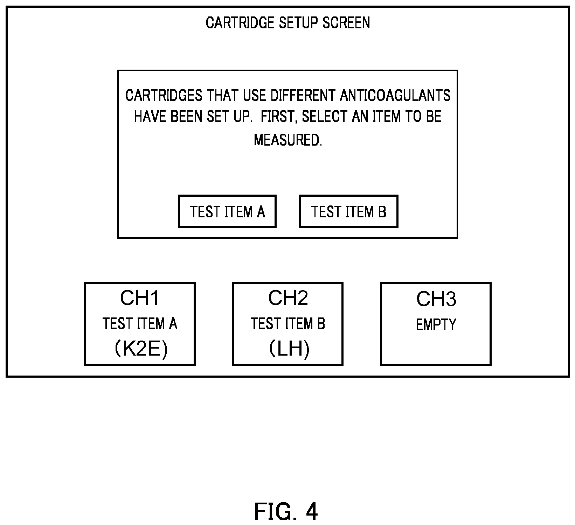

A second aspect will be described with reference to FIG. 4. In the second aspect, a case in which the user intends to test plural test items ("test item A" and "test item B") will be described. Note that test cartridge 180a is test cartridge 180 used to check test item A and it is assumed that only K2E is usable for test item A. On the other hand, test cartridge 180b is test cartridge 180 used to check test item B and it is assumed that only LH is usable for test item B. It is assumed that the user has separately prepared a blood sample containing K2E and a blood sample containing LH.

First, two test cartridges 180 (test cartridge 180a and test cartridge 180b) are set here in two cartridge loading sections 150, respectively. In so doing, blood testing device 100 recognizes the test items (test item A and test item B) in test cartridge 180a and test cartridge 180b based on bar codes or the like.

The second information is displayed on the touch panel display that combines input section 110 and output section 140. As shown in FIG 4, according to the present embodiment, test items A and B and the names of anticoagulants usable for the respective test items are each displayed on the touch panel display.

As described above, since test item A and test item B differ from each other in usable anticoagulants, blood testing device 100 allows the user to select which test item to test first. Note that according to the present embodiment, as shown in FIG. 4, control section 130 displays a pop-up or the like, notifying the user and allowing the user to select which test item to test first.

It is assumed here that the user selects test item A first. Next, the user dispenses a blood sample containing K2E into test cartridge 180a. Then, control section 130 conducts a blood test for test item A using the blood sample containing K2E and dispensed into test cartridge 180a.

It is assumed here that the user selects test item B next. After the blood test for test item A is finished, the user dispenses a blood sample containing LH into test cartridge 180b. Then, control section 130 conducts a blood test for test item B using the blood sample containing LH and dispensed into test cartridge 180b.

### (Third aspect)

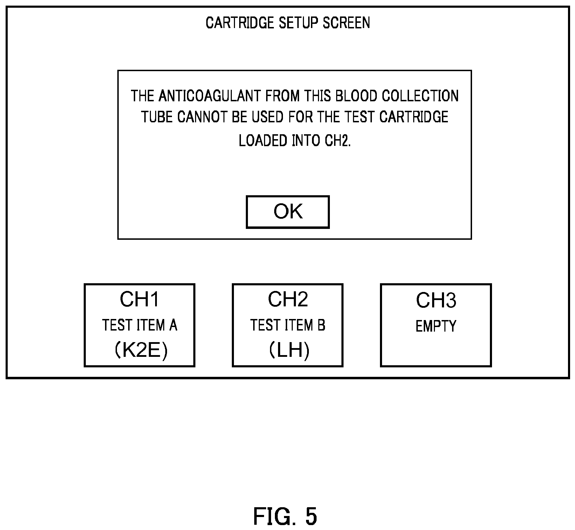

A third aspect will be described with reference to FIG. 5. Description will be given of a case in which control section 130 compares the second information entered via input section 110 with the first information, determines whether the anticoagulant contained in the blood collection tube is usable for testing a specific test item, and then performs control to make the test item in question unselectable, notifies that the test item in question cannot be tested, or performs control to disable the test item in question from being tested if it is determined that the anticoagulant contained in the blood collection tube is unusable. Note that test cartridge 180a is test cartridge 180 used to check test item A and it is assumed that only K2E is usable for test item A. On the other hand, test cartridge 180b is test cartridge 180 used to check test item B and it is assumed that only LH is usable for test item B. In this way, the first information about the anticoagulants usable for respective test items is shown on the touch panel display.

First, the bar code on the blood collection tube to be tested is scanned to enter first information a. First information a contains information about anticoagulant K2E. Next, two test cartridges 180 (test cartridge 180a and test cartridge 180b) are set here in two cartridge loading sections 150, respectively.

In so doing, control section 130 notifies that the blood sample including entered first information a cannot be used in blood testing for test item B for which test cartridge 180b is used. Regarding a method for notification, a pop-up may be displayed newly or a warning sound may be used for notification. Also, control section 130 performs control not to check the test item for which test cartridge 180b is used. Specifically, control is performed not to allow test item B to be entered even if the area with test item B indicated thereon is touched. Furthermore, control section 130 may be kept from testing test item B even if test item B is selected. Specifically, even if the area with test item B indicated thereon is touched, control section 130 does not transmit a signal for testing test item B to measuring section 160.

Next, the user dispenses a blood sample only into test cartridge 180a. Then, control section 130 conducts a blood test for test item A for which test cartridge 180a is used.

As described above, since the first information about usable anticoagulants or unusable anticoagulants for respective test items is output, blood testing device 100 according the present embodiment can prevent the user from mistakenly conducting a blood test using a blood sample containing an anticoagulant unusable for a specific test item.

Note that although in the example described above, the first information is stored in storage section 120, the first information may be stored externally. In that case, the first information may be acquired for each blood test or acquired periodically. Alternatively, the first information may be carried by test cartridge 180. In that case, the first information may be configured to be entered automatically or manually into blood testing device 100 when test cartridge 180 is set in cartridge loading section 150.

The present application claims priority from Japanese Patent Application No. 2017-115891, filed June 13, 2017. The contents described in the document and drawings are incorporated herein by reference.

### Industrial Applicability

The blood testing device according to the present invention does not test any test item using a blood sample containing any anticoagulant inappropriate for the test item. Thus, improvement in the reliability of blood testing results and further dissemination of blood testing are expected.

### Reference Signs List

100 Blood testing device
110 Input section
120 Storage section
130 Control section
140 Output section
150 Cartridge loading section
160 Measuring section
170 Casing
180, 180a, 180b Test cartridge

## Claims

1. A blood testing device for testing blood in a blood collection tube, the blood testing device comprising an output that externally outputs first information about usable anticoagulants or unusable anticoagulants for respective test items.

2. The blood testing device according to claim 1, further comprising:
an input used to enter second information about an anticoagulant contained in the blood collection tube; and
a controller that compares the second information entered via the input with the first information and determines whether the anticoagulant contained in the blood collection tube is usable for testing a specific test item, wherein
the output further externally outputs a determination result produced by the controller.

3. The blood testing device according to claim 2, wherein as information about each anticoagulant, the first information and the second information contain a name of the anticoagulant, a character code corresponding to the anticoagulant, a color code corresponding to the anticoagulant and marked on a cap of the blood collection tube, or a combination thereof.

4. The blood testing device according to claim 3, wherein the color code is a color code defined by JIS T 3233, ISO 6710, or CLSI GP41-A6.

5. The blood testing device according to claim 3, wherein the color code is a color code set arbitrarily for the anticoagulant.

6. The blood testing device according to any one of claims 2 to 4, wherein the output outputs the first information, or the first information and the determination result as visual information including at least either characters or graphics.

7. The blood testing device according to any one of claims 2 to 5, wherein the output outputs the first information, or the first information and the determination result as auditory information including voice information.

8. The blood testing device according to any one of claims 1 to 7, further comprising a storage section storing the first information.

9. The blood testing device according to any one of claims 1 to 7, wherein the first information is stored externally.

10. The blood testing device according to any one of claims 2 to 9, wherein, in testing a plurality of test items, when an anticoagulant contained in the blood collection tube is unusable for one or more of the plurality of test items, the controller performs control to make the test item in question unselectable, notifies that the test item in question is not testable, or performs control to disable the test item in question from being tested.
